(19) Europäisches Patentamt
European Patent Office
Office européen des brevets

(11) **EP 1 855 108 A1**

(12) **EUROPÄISCHE PATENTANMELDUNG**

(43) Veröffentlichungstag:
**14.11.2007 Patentblatt 2007/46**

(21) Anmeldenummer: 07009250.7

(22) Anmeldetag: **08.05.2007**

(51) Int Cl.:
*G01N 30/86* (2006.01)    *G01N 21/76* (2006.01)
*G01N 33/00* (2006.01)    *G01N 30/02* (2006.01)
*G01N 30/40* (2006.01)    *G01N 30/46* (2006.01)
*G01N 30/74* (2006.01)

(84) Benannte Vertragsstaaten:
**AT BE BG CH CY CZ DE DK EE ES FI FR GB GR HU IE IS IT LI LT LU LV MC MT NL PL PT RO SE SI SK TR**
Benannte Erstreckungsstaaten:
**AL BA HR MK YU**

(30) Priorität: **09.05.2006   DE 102006021619**

(71) Anmelder: **Linde AG**
**65189 Wiesbaden (DE)**

(72) Erfinder:
• **Uschner, Bernd**
  **80538 München (DE)**
• **Wenning, Ulrike**
  **82049 Pullach (DE)**

(54) **Verfahren und Vorrichtung zur Spurenanalytik von Stickstoffmonoxid in kohlenwasserstoffhaltigen Gasen**

(57)    Die Erfindung beschreibt ein Verfahren und eine Vorrichtung zur Spurenanalytik von Stickstoffmonoxid (NO) im Gasstrom von kohlenwasserstoffhaltigen Gasen in einer chemischen oder petrochemischen Anlage, insbesondere Olefinanlage oder Raffinerie, durch die Entnahme von gasförmigen Proben und deren Analyse mittels einer Gaschromatographen(GC)-Säulenschaltung, bestehend aus mindestens zwei GC-Säulen und einem Ventil, und eines Chemilumineszenzdetektor, wobei die Signale des Chemilumineszenzdetektors von mehreren nacheinander entnommenen Proben mittels eines Computerprogramms erfasst und akkumuliert werden.

EP 1 855 108 A1

**Beschreibung**

Die Erfindung betrifft ein Verfahren zur Spurenanalytik von Stickstoffmonoxid (NO) im Gasstrom von kohlenwasserstoffhaltigen Gasen in einer chemischen oder petrochemischen Anlage, insbesondere Olefinanlage und Raffinerie, durch die Entnahme gasförmiger Proben und deren Analyse mittels einer Gaschromatographie(GC)-Säulenschaltung, bestehend aus mindestens zwei GC-Säulen und einem Ventil, und eines Chemilumineszenzdetektors, sowie eine Vorrichtung zur Durchführung des Verfahrens.

Im Tieftemperaturteil (Cold-Box) von petrochemischen Anlagen ist Stickstoffmonoxid (NO) selbst in geringsten Spuren ein Sicherheitsrisiko. Bei den dort herrschenden tiefen Temperaturen reagiert NO mit dem immer in geringen Spuren vorhanden Sauerstoff zu $NO_2$. $NO_2$ reichert sich an und reagiert mit bestimmten Kohlenwasserstoffen in der Anlage (zum Beispiel Dienen) und Ammoniak zu instabilen und somit explosiven nitrosen Harzen und Salzen. Diese Stoffe haben in der Vergangenheit schon zu schwersten Unfällen geführt (zum Beispiel Cold-Box Explosion am Steamcracker der Shell in Berre, Frankreich 1990).

Bei dem bestehenden Verdacht, dass sich NO im Einsatzstrom des Tieftemperaturteils der petrochemischen Anlage befindet, muss daher die Anlage in gewissen Zeitintervallen prophylaktisch abgeschaltet werden, so dass der Tieftemperaturteil angewärmt und ausgewaschen werden kann, bevor sich eine kritische Menge der instabilen nitrosen Harze und Salze bildet. Wegen der durchzuführenden Reinigungsarbeiten und den damit verbundenen Produktionsausfall stellt diese Prozedur einen erheblichen Kostenfaktor da. Um diese Kosten zu minimieren, muss das Zeitintervall zwischen zwei Reinigungsprozeduren möglichst lang sein. Für eine derartige Anpassung der Zeitintervalle ist die präzise Kenntnis der geringsten NO-Spuren im Einsatzstrom unerlässlich, um zuverlässig die in den Tieftemperaturteil gelangte NO-Menge bestimmen zu können und so exakt den Zeitpunkt der nötigen Reinigung zu definieren.

Eine weitere Möglichkeit zur Vermeidung unnötiger Anlagenstillstände ist die Entfernung der Stickstoffmonoxide aus dem Einsatzstrom vor dem Tieftemperaturteil der Anlage. Der Erfolg und die Zuverlässigkeit einer NO-Entfernung kann nur mit Hilfe einer empfindlichen und zuverlässigen NO-Spurenanalytik überprüft werden. Ohne eine derartige Analytik kann ein solches Verfahren weder entwickelt noch in der Praxis eingesetzt werden.

Ein Verfahren und eine Vorrichtung zur Spurenanalyse von kleinsten NO-Mengen in kohlenwasserstoffhaltigen Gasen werden in der Patentanmeldung DE 102004055120A1 beschrieben. Bei der beschriebenen Methode werden eine GC-Säulenschaltung aus beispielsweise zwei gepackten GC-Säulen, ein Ventil und ein Chemilumineszenzdetektor (CLD) verwendet. Aus dem Einsatzstrom des Tieftemperaturteils wird eine gasförmige Probe in eine Probenschleife abgefüllt. In der ersten Phase der Analyse wird die Probe aus der Probenschleife mittels Stickstoff in die erste GC-Säule gedrückt. In dieser Säule werden die Kohlenwasserstoffe deutlich stärker zurückgehalten als beispielsweise NO, $CH_4$ und CO, welche nach einer gewissen Verweilzeit (Retentionszeit) als erstes aus der Säule austreten. Sobald das NO die erste Säule verlassen und die zweite Säule erreicht hat, wird ein Ventil umgeschaltet und die zweite Phase gestartet. Die erste Säule wird in umgekehrter Richtung mit Stickstoff durchspült, wodurch die in der ersten Phase adsorbierten Kohlenwasserstoffe wieder von der ersten Säule entfernt werden. Gleichzeitig werden NO, $CH_4$ und CO und andere leichte Komponenten mittels Stickstoff durch die zweite GC-Säule gespült und weiter aufgetrennt. Schließlich gelangt das NO zum CLD und wird dort detektiert. Nach dem Ende der Rückspülphase wird das Ventil wieder umgeschaltet, so dass eine neue Messung gestartet werden kann. Durch die so erfolgte Messung der NO-Konzentration im Einsatzstrom kann bei gleichzeitiger Bestimmung der Menge des Einsatzstromes, die NO-Menge bestimmt werden, die im Tieftemperaturteil der Anlage deponiert wird. Somit ist eine Reinigungsabschaltun g zu einem definierten Zeitpunkt nach dem Erreichen einer bestimmten NO-Menge im Tieftemperaturteil der Anlage möglich.

Die Nachteile des, in DE 102004055120A1 beschriebenen, Verfahrens liegen in der Datenanalyse. Bei einer einfachen Auswertung des Integrals oder der Signalamplitude mittels Vergleich mit einer Eichprobe definierter NO-Konzentration ist die Nachweisgrenze für die zu messende NO-Konzentration durch das gemessene Signal (S) zu Rausch (R) Verhältnis limitiert und liegt bei wenigen ppb.

Der Erfindung liegt daher die Aufgabe zugrunde, die Nachweisgrenze des Verfahrens zu senken.

Diese Aufgabe wird verfahrensseitig dadurch gelöst, dass die Signale des Chemilumineszenzdetektors mittels eines Computerprogramms automatisch erfasst, die Signale mehrerer einzelner nacheinander erfolgter Messungen nacheinander entnommener Proben akkumuliert und die akkumulierten Signale zur Bestimmung der NO-Konzentration im Gasstrom ausgewertet werden.

Die erfindungsgemäße Akkumulation mehrerer einzelner nacheinander erfolgender Messungen (n) eines Gases wird das Signal zu Rausch Verhältnis analog zur Beziehung

$$S/N \propto \sqrt{n}$$

deutlich verbessert. Dadurch wird die Detektion deutlich kleinerer Signale möglich und so die Nachweisgrenze für die NO-Konzentration gesenkt. Schon die vierfache Akkumulation verbessert das Signal zu Rausch Verhältnis um den Faktor zwei, wodurch die Nachweisgrenze für die Bestimmung der NO-Konzentration um die Hälfte gesenkt wird.

Die Akkumulation von Signalen zur Verbesserung des Signal zu Rausch Verhältnisses ist bei verschiedenen Analyseverfahren wie bei der Infrarotspektroskopie oder Kernspinresonanzspektroskopie Stand der Technik. Bei Gaschromatographieanalysen ist die Akkumulation dagegen jedoch neu, da eine Gaschromatographieanalyse nach dem Stand der Technik zwischen mehreren Minuten und einer Stunde dauert. Bei der mehrmaligen Wiederholung von derartig langen Einzelanalysen kommt es zu Veränderungen der Zeit, die die Probe zum Durchlaufen einer GC-Säule braucht, (Retentionszeit) und zu damit einhergehenden Änderungen der Signalform. Die Konstanz von Retentionszeit und Signalform sind aber Vorraussetzungen für die Aufnahme und Analyse von akkumulierten Signalen. Abgesehen davon würde eine Wiederholung derartig langer Einzelmessungen zu einer unpraktikablen Dauer der Gesamtanalyse führen.

In dem hier beschriebenen Verfahren erfolgt die Analyse einer einzelnen Probe relativ schnell (2 bis 4 Minuten), da die schweren Gaskomponenten mit längeren Retentionszeiten von der ersten Säule zurückgespült werden und die Probenschleife von der nächsten zu messenden Gasprobe durchströmt wird, sobald das NO die zweite Säule erreicht hat. Innerhalb dieses Zeitrahmens erweisen sich Retentionszeit und Signalform als hinreichend konstant für eine Akkumulation und die Gesamtanalysendauer bleibt innerhalb eines praktikablen Zeitrahmens.

Gemäß einer besonders bevorzugten Ausführungsform der Erfindung erfolgt die Bestimmung der NO-Konzentration durch den Vergleich des Integrals und / oder der Signalamplitude des akkumulierten Signals mit dem Integral und / oder der Signalamplitude eines Signals einer Probe mit definierter NO-Konzentration. Vorteilhafterweise wird der Akkumulationszeitraum immer der Zeit angepasst, die vom Eintreffen der Probe an der zweiten Säule bis zum Passieren des Detektors verstreicht, wobei der Zeitpunkt maximaler Signalamplitude als Bezugspunkt dient.

Durch die erfindungsgemäße Erfassung der Signale mittels eines Computerprogramms ist es möglich dem kohlenwasserstoffhaltigem Gas automatisch, unbemannt und permanent Proben zu entnehmen und zu analysieren. Ebenso ist es vorteilhaft die gesamte in den Tieftemperaturteil gelangte NO-Menge aus der NO-Konzentration und der Größe des jeweils in den Tieftemperaturteil geführten Gasstromes zu bestimmen und beim Erreichen eines voreingestellten Wertes für die NO-Menge einen Alarm und / oder die Abschaltung der Anlage auszulösen. In einer weiteren Ausgestaltung der Erfindung wird bei der wiederholten Bestimmung einer NO-Konzentration oberhalb eines voreingestellten Wertes ein Alarm und / oder die Abschaltung der Anlage ausgelöst, wobei die die Zahl der hierfür notwendigen Wiederholungen ebenfalls voreingestellt wird. Die Verwendung der akkumulierten und ausgewerteten Signale zur Steuerung von vor- und / oder nachgeschalteten Prozessen, insbesondere zur Temperatursteuerung eines Katalysators, der Schwefel- bzw. Schwefelwasserstoffkonzentration und / oder einer anderen Komponente im Gasstrom, ist ebenso wie die Verwendung des Signals zur Steuerung des Ventils, das zwischen den beiden Phasen einer Messung umschaltet, zweckmäßig.

Vorrichtungsseitig wird die gestellte Aufgabe dadurch gelöst, dass der Chemilumineszenzdetektor mit einer Einrichtung gekoppelt ist, die die Signale des Chemilumineszenzdetektors aus mehreren einzelnen nacheinander erfolgenden Messungen akkumulieren kann.

Gemäß einer besonders bevorzugten Ausführung der Erfindung weist die Einrichtung die Fähigkeit zur Integration und zur Bestimmung der Signalamplitude auf. Eine Einrichtung zur Auslösung eines Alarms und / oder zur Abschaltung der Anlage ist ebenso zweckmäßig.

Mit der vorliegenden Erfindung gelingt es insbesondere die Nachweisgrenze für die NO-Konzentration in kohlenwasserstoffhaltigen Gasen deutlich zu senken. Dadurch wird eine genauere Bestimmung, der in den Tieftemperaturteil der Anlage gelangten, NO-Menge möglich und der Zeitpunkt für eine nötige Reinigungsabschaltung kann somit genauer definiert werden, wodurch die Laufzeit der Anlage und ihre Wirtschaftlichkeit erhöht wird.

**Patentansprüche**

1. Verfahren zur Spurenanalytik von Stickstoffmonoxid (NO) im Gasstrom von kohlenwasserstoffhaltigen Gasen in einer chemischen oder petrochemischen Anlage, insbesondere Olefinanlage oder Raffinerie, durch die Entnahme von gasförmigen Proben und deren Analyse mittels einer Gaschromatographen (GC)-Säulenschaltung, bestehend aus mindestens zwei GC-Säulen und einem Ventil, und eines Chemilumineszenzdetektors, **dadurch gekennzeichnet, dass** die Signale des Chemilumineszenzdetektors mittels eines Computerprogramms automatisch erfasst, die Signale mehrerer einzelner nacheinander erfolgter Messungen nacheinander entnommener Proben akkumuliert und die akkumulierten Signale zur Bestimmung der NO-Konzentration im Gasstrom ausgewertet werden.

2. Verfahren nach Anspruch 1, **dadurch gekennzeichnet, dass** die NO-Konzentration durch den Vergleich des Integrals und / oder der Signalamplitude des akkumulierten Signals mit dem Integral und / oder der Signalamplitude eines Signals einer Probe definierter NO-Konzentration ermittelt wird.

3. Verfahren nach Anspruch 1 oder 2, **dadurch ge-**

**kennzeichnet, dass** der Zeitraum, innerhalb dessen die Signale akkumuliert werden, immer der Zeit angepasst wird, die vom Eintreffen des Stickstoffmonoxides an der zweiten GC-Säule bis zum Passieren des Detektors verstreicht, wobei als Bezugspunkt der Zeitpunkt maximaler Signalamplitude am Detektor dient.

4.  Verfahren nach einem der Ansprüche 1 bis 3, **dadurch gekennzeichnet, dass** dem kohlenwasserstoffhaltigem Gas automatisch, unbemannt und permanent Proben entnommen und analysiert werden.

5.  Verfahren nach einem der Ansprüche 1 bis 4, **dadurch gekennzeichnet, dass** kontinuierlich die gesamte in den Tieftemperaturteil gelangte NO-Menge aus der NO-Konzentration und der Größe des jeweils in den Tieftemperaturteil geführten Gasstromes bestimmt wird und bei dem Erreichen eines voreingestellten Wertes für die NO-Menge ein Alarm ausgelöst und / oder die Anlage abgeschaltet wird.

6.  Verfahren nach einem der Ansprüche 1 bis 5, **dadurch gekennzeichnet, dass** bei wiederholter Bestimmung einer NO-Konzentration oberhalb eines voreingestellten Wertes ein Alarm ausgelöst und / oder die Anlage abgeschaltet wird, wobei die Zahl der hierfür notwendigen Wiederholungen ebenfalls voreingestellt wird.

7.  Verfahren nach einem der Ansprüche 1 bis 6, **dadurch gekennzeichnet, dass** die akkumulierten und ausgewerteten Signale zur Steuerung von vor- und / oder nachgeschalteten Prozessen, insbesondere zur Temperatursteuerung eines Katalysators, der Schwefel- bzw. Schwefelwasserstoffkonzentration und / oder einer anderen Komponente im Gasstrom, dienen.

8.  Verfahren nach einem der Ansprüche 1 bis 7, **dadurch gekennzeichnet, dass** die Signale zur Steuerung des Ventils, das zwischen den beiden Phasen einer Messung umschaltet, verwendet werden.

9.  Vorrichtung zur Spurenanalytik von Stickstoffmonoxid (NO) im Gasstrom von kohlenwasserstoffhaltigen Gasen in einer chemischen oder petrochemischen Anlage, insbesondere Olefinanlage oder Raffinerie, durch die Entnahme von gasförmigen Proben und deren Analyse mittels einer Gaschromatographen(GC)-Säulenschaltung, bestehend aus mindestens zwei GC-Säulen und einem Ventil, und eines Chemilumineszenzdetektor, **dadurch gekennzeichnet, dass** der Chemilumineszenzdetektor mit einer Einrichtung gekoppelt ist, die die Signale des Chemilumineszenzdetektors aus mehreren einzelnen nacheinander erfolgenden Messungen akkumulieren kann.

10.  Vorrichtung nach Anspruch 9, **dadurch gekennzeichnet, dass** die Einrichtung die Fähigkeit zur Integration und Bestimmung der Signalamplitude von Signalen aufweist.

11.  Vorrichtung nach Anspruch 9 oder 10, **dadurch gekennzeichnet, dass** die Vorrichtung eine Einrichtung zur Auslösung eines Alarms und / oder zur Abschaltung der Anlage aufweist.

EP 1 855 108 A1

**Europäisches Patentamt**

**EUROPÄISCHER RECHERCHENBERICHT**

Nummer der Anmeldung

EP 07 00 9250

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| Y,D | DE 10 2004 055120 A1 (LINDE AG [DE]) 6. April 2006 (2006-04-06) * Zusammenfassung * * Absatz [0002] * * Absatz [0023] - Absatz [0027] * ----- | 1-11 | INV. G01N30/86 G01N21/76 G01N33/00 |
| Y | US 6 100 096 A (BOLLINGER MARK J [US] ET AL) 8. August 2000 (2000-08-08) * Zusammenfassung * * Spalte 5, Zeile 54 - Spalte 6, Zeile 23 * ----- | 1-11 | ADD. G01N30/02 G01N30/40 G01N30/46 G01N30/74 |
| Y | GB 1 578 281 A (ANTEK INSTR) 5. November 1980 (1980-11-05) * Seite 2, Spalte 1, Zeile 53 - Spalte 2, Zeile 92 * * Seite 4, Spalte 1, Zeile 39 - Zeile 65 * * Seiten 1,2,4-6 * ----- | 2,10 | |
| Y | HALLE R T: "Potential Hazards of Nitrogen Oxide Compound Accumulations in Cryogenic Ethylene Recovery Facilities" PROCEEDINGS - ETHYLENE PRODUCERS CONFERENCE, AMERICAN INSTITUTE OF CHEMICAL ENGINEERS, NEW YORK, NY, US, Bd. 5, 1994, Seiten 120-182, XP002356003 ISSN: 1066-1557 * Seite 160, Absatz 4 * ----- | 4 | RECHERCHIERTE SACHGEBIETE (IPC) G01N |
| Y | EP 0 511 729 A2 (THERMEDICS INC [US] THERMEDICS DETECTION INC [US]) 4. November 1992 (1992-11-04) * Zusammenfassung * * Spalte 9, Zeile 40 - Spalte 10, Zeile 20 * * Abbildung 4 * ----- | 5,6,11 | |

-/--

Der vorliegende Recherchenbericht wurde für alle Patentansprüche erstellt

| Recherchenort | Abschlußdatum der Recherche | Prüfer |
|---|---|---|
| München | 5. Juli 2007 | Bravin, Michel |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
& : Mitglied der gleichen Patentfamilie, übereinstimmendes Dokument

5

**Europäisches Patentamt**

# EUROPÄISCHER RECHERCHENBERICHT

Nummer der Anmeldung

EP 07 00 9250

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| Y | US 2004/099046 A1 (MUELLER FRIEDHELM [DE]) 27. Mai 2004 (2004-05-27) <br> * Absätze [0024], [0029] * <br> * Abbildung 4 * <br> ----- | 4,7,8 | |
| Y | US 2005/106737 A1 (KISHKOVICH OLEG P [US] ET AL) 19. Mai 2005 (2005-05-19) <br> * Zusammenfassung * <br> * Absatz [0037] * <br> * Absatz [0150] - Absatz [0152] * <br> ----- | 1,9 | |
| Y | US 4 875 169 A (SYNOVEC ROBERT E [US] ET AL) 17. Oktober 1989 (1989-10-17) <br> * Zusammenfassung; Abbildungen 2,3,6,8 * <br> ----- | 1,9 | |
| | | | **RECHERCHIERTE SACHGEBIETE (IPC)** |

Der vorliegende Recherchenbericht wurde für alle Patentansprüche erstellt

| Recherchenort | Abschlußdatum der Recherche | Prüfer |
|---|---|---|
| München | 5. Juli 2007 | Bravin, Michel |

KATEGORIE DER GENANNTEN DOKUMENTE

X : von besonderer Bedeutung allein betrachtet
Y : von besonderer Bedeutung in Verbindung mit einer
   anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
   nach dem Anmeldedatum veröffentlicht worden ist
D : in der Anmeldung angeführtes Dokument
L : aus anderen Gründen angeführtes Dokument
........................................................................
& : Mitglied der gleichen Patentfamilie, übereinstimmendes
   Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 07 00 9250

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

05-07-2007

| Im Recherchenbericht angeführtes Patentdokument | Datum der Veröffentlichung | Mitglied(er) der Patentfamilie | | Datum der Veröffentlichung |
|---|---|---|---|---|
| DE 102004055120 A1 | 06-04-2006 | EP | 1794105 A1 | 13-06-2007 |
| | | WO | 2006034766 A1 | 06-04-2006 |
| US 6100096 A | 08-08-2000 | US | 6635415 B1 | 21-10-2003 |
| GB 1578281 A | 05-11-1980 | KEINE | | |
| EP 0511729 A2 | 04-11-1992 | CA | 2060729 A1 | 21-09-1992 |
| | | DE | 69210676 D1 | 20-06-1996 |
| | | ES | 2090493 T3 | 16-10-1996 |
| US 2004099046 A1 | 27-05-2004 | WO | 02063290 A2 | 15-08-2002 |
| | | DE | 10105728 A1 | 05-09-2002 |
| | | EP | 1358477 A2 | 05-11-2003 |
| US 2005106737 A1 | 19-05-2005 | KEINE | | |
| US 4875169 A | 17-10-1989 | KEINE | | |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102004055120 A1

**In der Beschreibung aufgeführte Nicht-Patentliteratur**

- *Cold-Box Explosion am Steamcracker der Shell in Berre,* 1990